(19) Europäisches Patentamt
European Patent Office
Office européen des brevets

(11) Veröffentlichungsnummer: **0 014 881**
**B1**

(12) # EUROPÄISCHE PATENTSCHRIFT

(45) Veröffentlichungstag der Patentschrift: **17.02.82**

(51) Int. Cl.[3]: **C 07 D 273/04**, C 07 D 413/04, A 01 N 43/88

(21) Anmeldenummer: **80100543.0**

(22) Anmeldetag: **04.02.80**

(54) **Oxadiazinderivate, Verfahren zu ihrer Herstellung und ihre Verwendung als Schädlingsbekämpfungsmittel.**

(30) Priorität: **14.02.79 DE 2905687**

(43) Veröffentlichungstag der Anmeldung: **03.09.80 Patentblatt 80/18**

(45) Bekanntmachung des Hinweises auf die Patenterteilung: **17.02.82 Patentblatt 82/7**

(84) Benannte Vertragsstaaten: **AT BE CH DE FR GB IT NL SE**

(56) Entgegenhaltungen:
**DE-A-2 732 115**
**FR-A-2 283 133**

(73) Patentinhaber: **BAYER AG, Zentralbereich Patente, Marken und Lizenzen, D-5090 Leverkusen 1, Bayerwerk (DE)**

(72) Erfinder: **Sirrenberg, Wilhelm, Dr., Wuppertaler Strasse 21, D-4322 Sprockhövel (DE)**
Erfinder: **Klauke, Erich, Dr., Eichendorffweg 8, D-5068 Odenthal (DE)**
Erfinder: **Hammann, Ingeborg, Dr., Belfortstrasse 9, D-5000 Köln (DE)**
Erfinder: **Krehan, Ingomar, Dr., Ludwig-Jahn-Strasse 54, D-5000 Köln 40 (DE)**
Erfinder: **Stendel, Wilhelm, Dr., In den Birken 55, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Oxadiazinderivate, Verfahren zu ihrer Herstellung und ihre Verwendung als Schädlingsbekämpfungsmittel

Die Erfindung betrifft neue Oxadiazinderivate, ein Verfahren zu ihrer Herstellung und ihre Verwendung als Schädlingsbekämpfungsmittel, insbesondere als Insektizide.

Es ist bereits bekannt, daß bestimmte Oxadiazinderivate, wie z. B. 3-(3-Chlorophenyl)-6-(2,6-difluorophenyl)-3,4-dihydro-2H-1,3,5-oxadiazin-2,4-dion, insektizid wirksam sind (vergleiche DE-OS 2 732 115). Die Wirkung dieser Verbindungen ist jedoch, insbesondere bei niedrigen Wirkstoffkonzentrationen und Aufwandmengen nicht immer zufriedenstellend.

Es wurden nun neue Oxadiazinderivate der Formel I

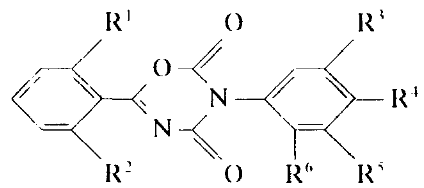

(I)

gefunden, in welcher

$R^1$ für Fluor, Chlor, Brom, Jod, Methyl oder Äthyl steht,

$R^2$ für Wasserstoff, Fluor, Chlor, Brom oder Jod steht,

$R^3$, $R^5$ und $R^6$ gleich oder verschieden sind und für Wasserstoff, Chlor, Methyl oder Trifluormethyl stehen,

$R^4$ für tert.-Butyl, tert.-Pentyl, Difluormethyl, Trifluormethyl, Chlordifluormethyl, Difluormethoxy, Trifluormethoxy, Chlordifluormethoxy, 1,1,2,2-Tetrafluoräthoxy, 2-Chlor-1,1,2-trifluoräthoxy, Trifluormethylthio, Chlordifluormethylthio oder zusammen mit $R^3$ für 2,2,4,4-Tetrafluor-1,3-dioxabutan-1,4-diyl, 2,2,3-Trifluor-1,4-dioxabutan-1,4-diyl, 2-Chlor-2,3,3-trifluor-1,4-dioxabutan-1,4-diyl, 2,2-Difluor-1,4-dioxa-butan-1,4-diyl oder 2,2-Difluor-1,3-dioxapropan-1,3-diyl steht und — für den Fall, daß wenigstens einer der Reste $R^3$, $R^5$ oder $R^6$ für Trifluormethyl steht — auch für Wasserstoff oder Chlor steht, sowie — für den Fall, daß $R^1$ für Chlor und gleichzeitig $R^2$ für Fluor steht — auch für Chlor, Methoxy, Äthoxy, Propoxy oder Butoxy steht.

Man erhält die Oxadiazinderivate der Formel I, wenn man substituierte Benzoylisocyanate der Formel II

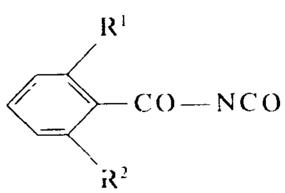

(II)

in welcher
$R^1$ und $R^2$ die oben angegebene Bedeutung haben,
mit substituierten Phenylisocyanaten der Formel III

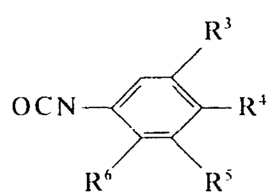

(III)

in welcher
$R^3$, $R^4$, $R^5$ und $R^6$ die oben angegebene Bedeutung haben,
gegebenenfalls unter Verwendung inerter Verdünnungsmittel umsetzt.

Überraschenderweise zeigen die neuen Oxadiazinderivate der Formel I eine erheblich höhere Wirksamkeit als Schädlingsbekämpfungsmittel, insbesondere durch eine höhere insektizide Wirkung als aus dem Stand der Technik bekannte Verbindungen analoger Konstitution und gleicher Wirkungsrichtung.

Verwendet man als Ausgangsstoffe beispielsweise 2-Chlorbenzoylisocyanat und 4-Trifluormethoxyphenylisocyanat, so kann die Reaktion dieser Verbindungen durch folgendes Formelschema skizziert werden:

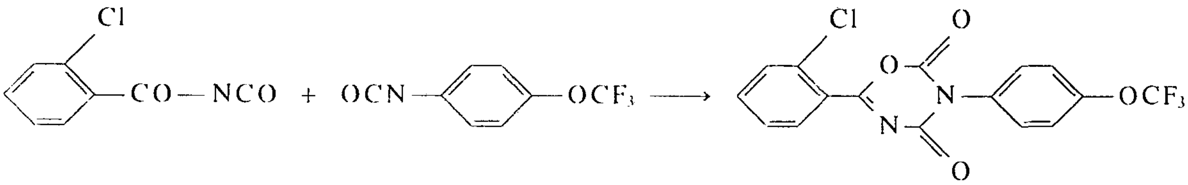

Die zu verwendenden Ausgangsstoffe sind durch die Formeln II und III definiert.

Die als Ausgangsverbindungen zu verwendenden Benzoylisocyanate der Formel II sind bekannt oder nach bekannten Verfahren herstellbar. Man erhält sie beispielsweise durch Umsetzung von substituierten Benzoesäureamiden mit Oxalylchlorid bei Temperaturen zwischen −20 und +100° C, gegebenenfalls unter Verwendung eines Verdünnungsmittels wie z. B. 1,2-Dichloräthan (vergleiche DE-OS 2 732 115). Als Beispiele für die Benzoylisocyanate der Formel II seien genannt:

2-Fluor-, 2-Chlor-, 2-Brom und 2-Jod-benzoylisocyanat sowie
2,6-Difluor-, 2,6-Dichlor- und 2-Chlor-6-fluorbenzoylisocyanat.

Die als weitere Ausgangsstoffe zu verwendenden substituierten Phenylisocyanate der Formel III sind ebenfalls bekannt oder nach bekannten Verfahren herstellbar (vergleiche z. B. J. Org. Chem. 29 (1964), 1−11; DE-OS 2 601 780 und 2 637 947). Als Beispiele für die substituierten Phenylisocyanate der Formel III seien genannt:

2-Chlor-, 3-Chlor- und 4-Chlor-phenylisocyanat, 3,4-Dichlor-, 2,4-Dichlor-,
3,5-Dichlor- und 2-Methyl-4-chlorphenylisocyanat, 4-tert.-Butyl- und
4-tert.-Pentylphenylisocyanat, 4-Methoxy-, 4-Äthoxy-, 4-Propoxy- und
4-Butoxy-phenylisocyanat, 3-Difluormethyl- und 4-Difluormethyl-phenylisocyanat,
3-Trifluormethyl- und 4-Trifluormethyl-phenylisocyanat, 3-Chlordifluormethyl- und
4-Chlordifluormethyl-phenylisocyanat, 3,5-Bis-trifluormethyl-phenylisocyanat,
3-Chlor-4-trifluormethyl-, 2-Chlor-4-trifluormethyl-, 4-Chlor-2-trifluormethyl-,
2-Chlor-5-trifluormethyl-, 2-Chlor-4-chlordifluormethyl- und
3-Chlor-4-chlordifluormethyl-phenylisocyanat, 4-Difluormethoxy- und
3-Chlor-4-difluormethoxy-phenylisocyanat, 4-Trifluormethoxy- und
3-Chlor-4-trifluormethoxy-phenylisocyanat, 4-Chlordifluormethoxy- und
3-Chlor-4-chlordifluormethoxy-phenylisocyanat, 4-(1,1,2,2-tetrafluoräthoxy)- und
4-(2-Chlor-1,1,2-trifluoräthoxy)-phenylisocyanat, 4-Trifluormethylthio-,
4-Chlordifluormethylthio-, 3-Chlor-4-trifluormethylthio- und
3-Chlor-4-chlordifluormethylthio-phenylisocyanat sowie
6-Isocyanato-2,2,4,4-tetrafluor-benzo-1,3-dioxin.

Das Verfahren zur Herstellung der neuen Oxadiazinderivate wird gegebenenfalls unter Verwendung inerter Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile, wie Acetonitril und Propionitril. Die Verwendung von Verdünnungsmitteln ist jedoch im allgemeinen nicht erforderlich.

Die Reaktionstemperatur kann innerhalb eines größeren Bereichs variiert werden. Im allgemeinen arbeitet man zwischen 20 und 200° C, vorzugsweise bei 50 bis 150° C. Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Komponenten werden, gegebenenfalls in einem geeigneten Verdünnungsmittel vermischt und mehrere Stunden bei der erforderlichen Temperatur gerührt. Nach dem Abkühlen werden die in fester Form anfallenden Produkte mit einem unpolaren Verdünnungsmittel wie z. B. Cyclohexan verrieben und durch Filtration abgetrennt. Zur Charakterisierung dient der Schmelzpunkt.

Die neuen Oxadiazinderivate zeichnen sich durch hervorragende Wirksamkeit bei der Bekämpfung von Schädlingen, insbesondere durch insektizide Wirksamkeit aus. Sie wirken gegen pflanzenschädigende Insekten, gegen Hygiene- und Vorratsschädlinge und gegen Ektoparasiten. Einige der neuen Verbindungen sind auch fungizid wirksam. Daher können die erfindungsgemäßen Verbindungen mit Erfolg im Pflanzenschutz, im Hygiene- und Vorratsschutz sowie im Veterinärbereich als Schädlingsbekämpfungsmittel verwendet werden.

Die Wirkstoffe eignen sich bei guter Pflanzenverträglichkeit und günstiger Warmblütertoxizität zur

Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregària.
Aus der Ordnung der Dermaptera z. B. Forficula auricularia.
Aus der Ordnung der Isoptera z. B. Reticulitermes spp.
Aus der Ordnung der Anoplura z. B. Phylloxera vastatrix, Pemphigus spp., Pediculus humanus corporis, Haematopinus spp., Linognathus spp.
Aus der Ordnung der Mallophaga z. B. Trichodectes spp., Damalinea spp.
Aus der Ordnung der Thysanoptera z. B. Hercinothrips femoralis, Thrips tabaci.
Aus der Ordnung der Heteroptera z. B. Eurygaster spp., Dysdercus intermedius, Piesma quadrata, Cimex lectularius, Rhodnius prolixus, Triatoma spp.
Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attangenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp.
Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z. B. Xenopsylla cheopis, Ceratophyllus spp.
Aus der Ordnung der Arachnida z. B. Scorpio maurus, Latrodectus mactans.
Aus der Ordnung der Acarina z. B. Acarus siro, Argas spp., Ornithodoros spp., Dermanyssus gallinac, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die Wirkstoffe können in die üblichen Formulierungen übergeführt werden, wie Lösungen, Emulsionen, Suspensionen, Pulver, Schäume, Pasten, Granulate, Aerosole, wirkstoffimprägnierte Natur- und synthetische Stoffe, Feinstverkapselungen in polymeren Stoffen und in Hüllmassen für

Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

## Beispiel A

### Plutella-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z. B. die Verbindungen der folgenden Herstellungsbeispiele eine besonders günstige Wirksamkeit: 1, 2, 16, 17, 18, 19, 21, 22, 24, 37, 38, 39, 41, 44, 45 und 46.

## Beispiel B

### Laphygma-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigt z. B. die Verbindung des Herstellungsbeispiels 15 eine besonders günstige Wirksamkeit.

## Beispiel C

### Mückenlarven-Test

Testtiere: Aedes aegypti
Lösungsmittel: Aceton
Emulgator: Gewichtsteile

Zur Herstellung einer zweckmäßigen Wirkstoffzubereitung löst man 2 Gewichtsteile Wirkstoff in 1000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wäßrigen Wirkstoffzubereitungen der gewünschten Konzentration in Gläser und setzt anschließend etwa 25 Mückenlarven in jedes Glas ein.

Nach 24 Stunden wird der Abtötungsgrad in % bestimmt. Dabei bedeutet 100%, daß alle Larven abgetötet worden sind. 0% bedeutet, daß überhaupt keine Larven abgetötet worden sind.

Bei diesem Test zeigen z. B. die Verbindungen der folgenden Herstellungsbeispiele eine besonders günstige Wirksamkeit: 37, 38 und 46.

## Beispiel D

### Test mit Lucilia cuprina res.-Larven

Emulgator: 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen z. B. die Verbindungen der folgenden Herstellungsbeispiele eine besonders günstige Wirksamkeit: 15, 16, 37 und 41.

## Herstellungsbeispiele

### Beispiel 1

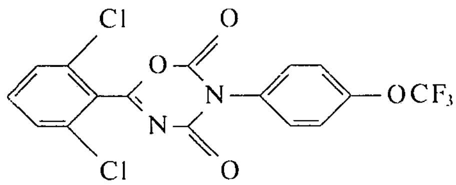

Unter Ausschluß von Feuchtigkeit werden 6,48 g (0,03 Mol) 2,6-Dichlorbenzoylisocyanat und 6,09 g (0,03 Mol) 4-Trifluormethoxyphenylisocyanat 16 Stunden lang auf 100–110°C erhitzt. Nach dem Abkühlen wird das als kompakte Kristallmasse anfallende Reaktionsprodukt sorgfältig mit Cyclohexan verrieben, abgesaugt, je einmal mit Cyclohexan und anschließend mit Petroläther gewaschen und dann getrocknet. Man erhält 12,2 g (97% der Theorie) der kristallinen Verbindung mit einem Schmelzpunkt von 208°C.

Entsprechend dem Beispiel 1 wurden die folgenden Verbindungen der allgemeinen Formel I

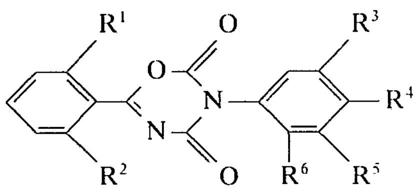

(I)

hergestellt (die Ausbeuten wurden nicht optimiert):

| Beispiel Nr. | $R^1$ | $R^2$ | $R^3$ | $R^4$ | $R^5$ | $R^6$ | Ausbeute (% der Theorie) | Schmelzpunkt (°C) |
|---|---|---|---|---|---|---|---|---|
| 2 | Cl | Cl | H | $SCF_3$ | H | H | 77,6 | 200 |
| 3 | Cl | Cl | $-CF_2-O-CF_2-O-$ | | H | H | 26,8 | 186 |
| 4 | Cl | Cl | H | $OCF_2CHF_2$ | H | H | 100 | 210 |
| 5 | Cl | Cl | H | $CHF_2$ | H | H | 75,5 | 213 |
| 6 | Cl | Cl | Cl | $SCF_3$ | H | H | 95,5 | 186 |
| 7 | Cl | Cl | Cl | $SCF_2Cl$ | H | H | 77 | 172 |
| 8 | Cl | Cl | Cl | $CF_2Cl$ | H | H | 84,5 | 208 |
| 9 | Cl | Cl | Cl | $OCF_3$ | H | H | 26,5 | 180 |
| 10 | Cl | Cl | $CF_3$ | H | H | H | 50,5 | 177 |
| 11 | Cl | Cl | Cl | $OCF_2Cl$ | H | H | 91,5 | 173 |
| 12 | Cl | Cl | H | $CF_2Cl$ | H | H | 89,5 | 214 |
| 13 | Cl | Cl | H | $OCF_2Cl$ | H | H | 92,5 | 198 |
| 14 | Cl | Cl | $CF_3$ | H | $CF_3$ | H | 96,5 | 203 |
| 15 | Cl | H | H | $OCF_3$ | H | H | 92,5 | 205 |
| 16 | Cl | H | H | $SCF_3$ | H | H | 80 | 196 |

Fortsetzung

| Beispiel Nr. | $R^1$ | $R^2$ | $R^3$ | $R^4$ | $R^5$ | $R^6$ | Ausbeute (% der Theorie) | Schmelzpunkt (°C) |
|---|---|---|---|---|---|---|---|---|
| 17 | Cl | H | $-CF_2-O-CF_2-O-$ | | H | H | 69,5 | 197 |
| 18 | Cl | H | Cl | $OCHF_2$ | H | H | 51 | 172 |
| 19 | Cl | H | H | $OCF_2-CHF_2$ | H | H | 86,5 | 203 |
| 20 | Cl | H | H | $CHF_2$ | H | H | 78 | 200 |
| 21 | Cl | H | Cl | $SCF_3$ | H | H | 37,9 | 153 |
| 22 | Cl | H | Cl | $SCF_2Cl$ | H | H | 57,5 | 153 |
| 23 | Cl | H | Cl | $CF_2Cl$ | H | H | 26,5 | 184 |
| 24 | Cl | H | Cl | $OCF_3$ | H | H | 72,5 | 167 |
| 25 | Cl | H | Cl | $OCF_2Cl$ | H | H | 71 | 155 |
| 26 | Cl | H | H | $CF_2Cl$ | H | H | 83 | 215 |
| 27 | Cl | H | H | $OCHF_2$ | H | H | 91 | 195 |
| 28 | Cl | H | H | $OCF_2Cl$ | H | H | 83,5 | 198 |
| 29 | Cl | H | H | $SCF_2Cl$ | H | H | 78 | 187 |
| 30 | Cl | H | $CF_3$ | H | $CF_3$ | H | 50 | 188 |
| 31 | Cl | H | $CF_3$ | H | H | Cl | 28,5 | 179 |
| 32 | Cl | H | H | Cl | H | $CF_3$ | 33 | 166 |
| 33 | Cl | H | H | $CF_3$ | H | H | 81,5 | 213 |
| 34 | Cl | H | Cl | $CF_3$ | H | H | 76,5 | 194 |
| 35 | Cl | H | H | $CF_3$ | H | Cl | 31 | 174 |
| 36 | Cl | H | H | $CF_2Cl$ | H | Cl | 28,5 | 163 |
| 37 | Cl | F | H | $OCF_3$ | H | H | 63 | 199 |
| 38 | Cl | F | H | $SCF_3$ | H | H | 60,5 | 189 |
| 39 | Cl | F | $-CF_2-O-CF_2-O-$ | | H | H | 35,5 | 164 |
| 40 | Cl | F | Cl | $OCHF_2$ | H | H | 34,5 | 173 |
| 41 | Cl | F | H | $OCF_2-CHF_2$ | H | H | 85,5 | 216 |
| 42 | Cl | F | H | $CHF_2$ | H | H | 100 | 212 |
| 43 | Cl | F | Cl | $SCF_3$ | H | H | 17,5 | 162 |
| 44 | Cl | F | Cl | $SCF_2Cl$ | H | H | 23 | 141 |
| 45 | Cl | F | Cl | $CF_2Cl$ | H | H | 44,5 | 193 |

Fortsetzung

| Beispiel Nr. | $R^1$ | $R^2$ | $R^3$ | $R^4$ | $R^5$ | $R^6$ | Ausbeute (% der Theorie) | Schmelzpunkt (°C) |
|---|---|---|---|---|---|---|---|---|
| 46 | Cl | F | Cl | $OCF_3$ | H | H | 26 | 175 |
| 47 | Cl | F | Cl | Cl | H | H | 52 | 191 |
| 48 | Cl | F | Cl | Cl | H | H | 51,5 | 177 |
| 49 | Cl | F | H | $OCH_3$ | H | H | 66,5 | 172 |
| 50 | Cl | F | H | $C(CH_3)_3$ | H | H | 36,5 | 163 |
| 51 | Cl | F | $CF_3$ | H | H | H | 25,5 | 134 |
| 52 | Cl | F | Cl | $OCF_2Cl$ | H | H | 55 | 173 |
| 53 | Cl | F | H | $CF_2Cl$ | H | H | 57 | 217 |
| 54 | Cl | F | H | $OCHF_2$ | H | H | 73 | 182 |
| 55 | Cl | F | H | $OCF_2Cl$ | H | H | 87,5 | 202 |
| 56 | Cl | F | H | $SCF_2Cl$ | H | H | 42,5 | 186 |
| 57 | Cl | F | $CF_3$ | H | $CF_3$ | H | 30,5 | 191 |
| 58 | Cl | F | H | Cl | H | $CH_3$ | 31 | 179 |
| 59 | Cl | F | H | $CF_3$ | H | H | 63 | 221 |
| 60 | Cl | F | Cl | $CF_3$ | H | H | 51,5 | 202 |
| 61 | Cl | F | H | $CF_3$ | H | Cl | 21 | 185 |
| 62 | Cl | F | H | $CF_2Cl$ | H | Cl | 18 | 196 |
| 63 | F | F | H | $OCF_3$ | H | H | 67 | 194 |
| 64 | F | F | H | $SCF_3$ | H | H | 64,5 | 190 |
| 65 | F | F | $-CF_2-O-CF_2-O-$ | | H | H | 75 | 178 |
| 66 | F | F | H | $OCF_2CHF_2$ | H | H | 82 | 201 |
| 67 | F | F | H | $CHF_2$ | H | H | 68 | 203 |
| 68 | F | F | Cl | $SCF_3$ | H | H | 52,5 | 171 |
| 69 | F | F | Cl | $SCF_2Cl$ | H | H | 50,5 | 160 |
| 70 | F | F | Cl | $OCF_3$ | H | H | 76 | 190 |
| 71 | F | F | $CF_3$ | H | H | H | 58 | 146 |
| 72 | F | F | Cl | $OCF_2Cl$ | H | H | 80 | 180 |
| 73 | F | F | H | $CF_2Cl$ | H | H | 76 | 212 |
| 74 | F | F | H | $OCHF_2$ | H | H | 92 | 205 |

Fortsetzung

| Bei-spiel Nr. | $R^1$ | $R^2$ | $R^3$ | $R^4$ | $R^5$ | $R^6$ | Ausbeute (% der Theorie) | Schmelz-punkt (°C) |
|---|---|---|---|---|---|---|---|---|
| 75 | F | F | H | $OCF_2Cl$ | H | H | 77 | 184 |
| 76 | F | F | H | $SCF_2Cl$ | H | H | 67,5 | 182 |
| 77 | F | F | $CF_3$ | H | $CF_3$ | H | 45,5 | 175 |
| 78 | F | F | $CF_3$ | H | H | Cl | 59,5 | 185 |
| 79 | F | F | H | Cl | H | $CF_3$ | 35 | 166 |
| 80 | F | F | H | $CF_3$ | H | H | 64,5 | 230 (Z) |
| 81 | F | F | Cl | $CF_3$ | H | H | 53 | 204 |
| 82 | F | F | H | $CF_3$ | H | Cl | 22 | 181 |
| 83 | F | F | H | $CF_2Cl$ | H | Cl | 22,5 | 184 |
| 84 | Br | H | H | $OCF_3$ | H | H | 83 | 194 |
| 85 | Br | H | H | $SCF_3$ | H | H | 94 | 194 |
| 86 | Br | H | $-CF_2-O-CF_2-O-$ | | H | H | 86 | 192 |
| 87 | Br | H | H | $OCF_2CHF_2$ | H | H | 92 | 195 |
| 88 | Br | H | H | $CHF_2$ | H | H | 91 | 198 |
| 89 | Br | H | Cl | $SCF_3$ | H | H | 32,5 | 161 |
| 90 | Br | H | Cl | $SCF_2Cl$ | H | H | 62 | 146 |
| 91 | Br | H | Cl | $OCF_3$ | H | H | 85 | 161 |
| 92 | Br | H | $CF_3$ | H | H | H | 85 | 162 |
| 93 | Br | H | Cl | $OCF_2Cl$ | H | H | 68,5 | 172 |
| 94 | Br | H | H | $CF_2Cl$ | H | H | 73,5 | 214 |
| 95 | Br | H | H | $OCHF_2$ | H | H | 75 | 186 |
| 96 | Br | H | H | $OCF_2Cl$ | H | H | 75 | 195 |
| 97 | Br | H | H | $SCF_2Cl$ | H | H | 66 | 181 |
| 98 | Br | H | $CF_3$ | H | H | Cl | 36,5 | 185 |
| 99 | Br | H | H | $CF_3$ | H | H | 71 | 211 |
| 100 | Br | H | Cl | $CF_3$ | H | H | 65,5 | 192 |
| 101 | Br | H | H | $CF_3$ | H | Cl | 24,5 | 184 |
| 102 | Br | H | H | $CF_2Cl$ | H | Cl | 29 | 178 |
| 103 | J | H | H | $OCF_3$ | H | H | 95 | 174 |

Fortsetzung

| Beispiel Nr. | $R^1$ | $R^2$ | $R^3$ | $R^4$ | $R^5$ | $R^6$ | Ausbeute (% der Theorie) | Schmelzpunkt (°C) |
|---|---|---|---|---|---|---|---|---|
| 104 | J | H | H | $SCF_3$ | H | H | 88,5 | 177 |
| 105 | J | H | $-CF_2-O-CF_2-O-$ | | H | H | 91 | 184 |
| 106 | J | H | H | $OCF_2-CHF_2$ | H | H | 82,5 | 187 |
| 107 | J | H | H | $CHF_2$ | H | H | 61 | 189 |
| 108 | J | H | Cl | $SCF_3$ | H | H | 80,5 | 189 |
| 109 | J | H | Cl | $SCF_2Cl$ | H | H | 87 | 168 |
| 110 | J | H | Cl | $CF_2Cl$ | H | H | 91,5 | 186 |
| 111 | J | H | Cl | $OCF_3$ | H | H | 85 | 189 |
| 112 | J | H | $CF_3$ | H | H | H | 91,5 | 170 |
| 113 | J | H | Cl | $OCF_2Cl$ | H | H | 77,5 | 173 |
| 114 | J | H | H | $CF_2Cl$ | H | H | 69 | 202 |
| 115 | J | H | H | $OCHF_2$ | H | H | 81,5 | 152 |
| 116 | J | H | H | $OCF_2Cl$ | H | H | 72 | 180 |
| 117 | J | H | H | $SCF_2Cl$ | H | H | 57 | 162 |
| 118 | J | H | $CF_3$ | H | $CF_3$ | H | 72,5 | 194 |
| 119 | J | H | $CF_3$ | H | H | Cl | 48,5 | 178 |
| 120 | J | H | H | Cl | H | $CF_3$ | 28 | 141 |
| 121 | J | H | H | $CF_3$ | H | H | 70,5 | 192 |
| 122 | J | H | Cl | $CF_3$ | H | H | 75 | 194 |
| 123 | $CH_3$ | H | H | $OCF_3$ | H | H | 87,5 | 211 |
| 124 | $CH_3$ | H | H | $SCF_3$ | H | H | 81,5 | 203 |
| 125 | $CH_3$ | H | Cl | $SCF_3$ | H | H | 83,5 | 157 |
| 126 | $CH_3$ | H | Cl | $OCF_3$ | H | H | 79 | 170 |
| 127 | $CH_3$ | H | Cl | $OCF_2Cl$ | H | H | 68,5 | 162 |
| 128 | $CH_3$ | H | H | $CF_2Cl$ | H | H | 65,5 | 216 |
| 129 | $CH_3$ | H | H | $OCHF_2$ | H | H | 74,5 | 179 |
| 130 | $CH_3$ | H | H | $OCF_2Cl$ | H | H | 73,5 | 204 |
| 131 | $CH_3$ | H | H | $SCF_2Cl$ | H | H | 69 | 191 |
| 132 | $CH_3$ | H | H | $CF_3$ | H | H | 38,5 | 179 |

Fortsetzung

| Bei-spiel Nr. | $R^1$ | $R^2$ | $R^3$ | $R^4$ | $R^5$ | $R^6$ | Ausbeute (% der Theorie) | Schmelz-punkt (°C) |
|---|---|---|---|---|---|---|---|---|
| 133 | $CH_3$ | H | H | $CF_3$ | H | Cl | 41,5 | 180 |
| 134 | $CH_3$ | H | H | $CF_2Cl$ | H | Cl | 38,5 | 147 |
| 135 | $C_2H_5$ | H | H | $OCF_3$ | H | H | 21 | 145 |
| 136 | $C_2H_5$ | H | H | $SCF_3$ | H | H | 8,5 | 140 |
| 137 | $C_2H_5$ | H | $CF_3$ | H | H | H | 17,5 | 132 |
| 138 | $C_2H_5$ | H | Cl | $OCF_2Cl$ | H | H | 15 | 145 |
| 139 | $C_2H_5$ | H | H | $CF_2Cl$ | H | H | 19,5 | 183 |
| 140 | $C_2H_5$ | H | H | $OCHF_2$ | H | H | 19,5 | 164 |
| 141 | $C_2H_5$ | H | H | $OCF_2Cl$ | H | H | 20 | 143 |
| 142 | F | H | H | $OCF_3$ | H | H | 84 | 213 |
| 143 | F | H | H | $SCF_3$ | H | H | 63,5 | 197 |
| 144 | F | H | $-CF_2-O-CF_2-O-$ | | H | H | 95,5 | 189 |
| 145 | F | H | H | $OCF_2-CHF_2$ | H | H | 82 | 207 |
| 146 | F | H | H | $CHF_2$ | H | H | 74,5 | 218 |
| 147 | F | H | Cl | $SCF_3$ | H | H | 75,5 | 167 |
| 148 | F | H | Cl | $SCF_2Cl$ | H | H | 68,5 | 168 |
| 149 | F | H | Cl | $OCF_3$ | H | H | 83 | 178 |
| 150 | F | H | $CH_3$ | H | H | H | 65 | 142 |
| 151 | F | H | H | $CF_2Cl$ | H | H | 69 | 216 |
| 152 | F | H | H | $OCF_2Cl$ | H | H | 92 | 203 |
| 153 | F | H | H | $SCF_2Cl$ | H | H | 85 | 202 |
| 154 | F | H | $CF_3$ | H | $CF_3$ | H | 85,5 | 181 |
| 155 | F | H | $CF_3$ | H | H | Cl | 53 | 110 |
| 156 | F | H | H | $CF_3$ | H | H | 89 | 249 (Z) |

**Patentansprüche**

1. Oxadiazinderivate der Formel I

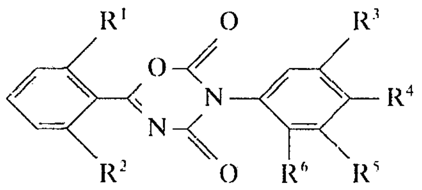

(I)

in welcher

$R^1$ für Fluor, Chlor, Brom, Jod, Methyl oder Äthyl steht,
$R^2$ für Wasserstoff, Fluor, Chlor, Brom oder Jod steht,
$R^3$, $R^5$ und $R^6$ gleich oder verschieden sind und für Wasserstoff, Chlor, Methyl oder Trifluormethyl stehen,
$R^4$ für tert.-Butyl, tert.-Pentyl, Difluormethyl, Trifluormethyl, Chlordifluormethyl, Difluormethoxy, Trifluormethoxy, Chlordifluormethoxy, 1,1,2,2-Tetrafluoräthoxy, 2-Chlor-1,1,2-trifluoräthoxy, Trifluormethylthio, Chlordifluormethylthio oder zusammen mit $R^3$ für 2,2,4,4-Tetrafluor-1,3-dioxabutan-1,4-diyl, 2,2,3-Trifluor-1,4-dioxabutan-1,4-diyl, 2-Chlor-2,3,3-trifluor-1,4-dioxabutan-1,4-diyl, 2,2-Difluor-1,4-dioxa-butan-1,4-diyl oder 2,2-Difluor-1,3-dioxapropan-1,3-diyl steht und — für den Fall, daß wenigstens einer der Reste $R^3$, $R^5$ oder $R^6$ für Trifluormethyl steht — auch für Wasserstoff oder Chlor steht, sowie — für den Fall, daß $R^1$ für Chlor und gleichzeitig $R^2$ für Fluor steht — auch für Chlor, Methoxy, Äthoxy, Propoxy oder Butoxy steht.

2. Verfahren zur Herstellung von Oxadiazinderivaten der Formel I

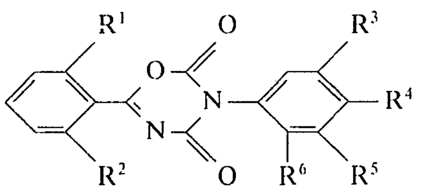

(I)

in welcher

$R^1$ für Fluor, Chlor, Brom, Jod, Methyl oder Äthyl steht,
$R^2$ für Wasserstoff, Fluor, Chlor, Brom oder Jod steht,
$R^3$, $R^5$ und $R^6$ gleich oder verschieden sind und für Wasserstoff, Chlor, Methyl oder Trifluormethyl stehen,
$R^4$ für tert.-Butyl, tert.-Pentyl, Difluormethyl, Trifluormethyl, Chlordifluormethyl, Difluormethoxy, Trifluormethoxy, Chlordifluormethoxy, 1,1,2,2-Tetrafluoräthoxy, 2-Chlor-1,1,2-trifluoräthoxy, Trifluormethylthio, Chlordifluormethylthio oder zusammen mit $R^3$ für 2,2,4,4-Tetrafluor-1,3-dioxabutan-1,4-diyl, 2,2,3-Trifluor-1,4-dioxabutan-1,4-diyl, 2-Chlor-2,3,3-trifluor-1,4-dioxabutan-1,4-diyl, 2,2-Difluor-1,4-dioxa-butan-1,4-diyl oder 2,2-Difluor-1,3-dioxapropan-1,3-diyl steht und — für den Fall, daß wenigstens einer der Reste $R^3$, $R^5$ oder $R^6$ für Trifluormethyl steht — auch für Wasserstoff oder Chlor steht, sowie — für den Fall, daß $R^1$ für Chlor und gleichzeitig $R^2$ für Fluor steht — auch für Chlor, Methoxy, Äthoxy, Propoxy oder Butoxy steht,

dadurch gekennzeichnet, daß man substituierte Benzoylisocyanate der Formel II

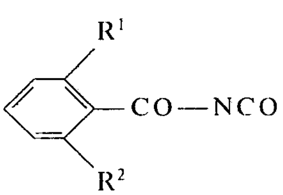

(II)

in welcher
$R^1$ und $R^2$ die oben angegebene Bedeutung haben,
mit substituierten Phenylisocyanaten der Formel III

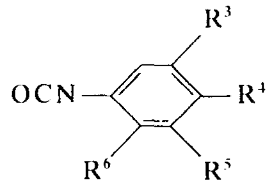

(III)

in welcher $R^3$, $R^4$, $R^5$ und $R^6$ die oben angegebene Bedeutung haben, gegebenenfalls unter Verwendung inerter Verdünnungsmittel umsetzt.

3. Schädlingsbekämpfungsmittel, gekennzeichnet durch einen Gehalt an Verbindungen der Formel I.

4. Insektizide nach Anspruch 3, gekennzeichnet durch einen Gehalt an Verbindungen der Formel I.

5. Verwendung von Verbindungen der Formel I zur Bekämpfung von Schädlingen, insbesondere von Insekten.

6. Verfahren zur Herstellung von Schädlingsbekämpfungsmitteln, dadurch gekennzeichnet, daß man Verbindungen der Formel I mit Streckmitteln und/oder oberflächenaktiven Mitteln mischt.

**Claims**

1. Oxadiazine derivatives of the formula I

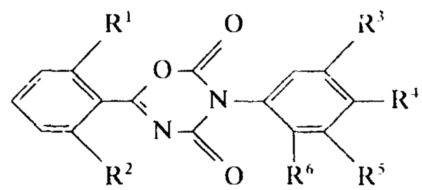

(I)

in which

$R^1$ represents fluorine, chlorine, bromine, iodine, methyl or ethyl,
$R^2$ represents hydrogen, fluorine, chlorine, bromine or iodine,
$R^3$, $R^5$ and $R^6$ are identical or different and represent hydrogen, chlorine, methyl or trifluormethyl and
$R^4$ represents tert.-butyl, tert.-pentyl, difluoromethyl, trifluoromethyl, chloridifluoromethyl, difluoromethoxy, trifluoromethoxy, chloridifluoromethoxy, 1,1,2,2-tetrafluoroethoxy, 2-chloro-1,1,2-trifluoroethoxy, trifluoromethylthio or chlorodifluoromethylthio, or, together with $R^3$, 2,2,4,4-tetrafluoro-1,3-dioxabutane-1,4-diyl, 2,2,3-trifluoro-1,4-dioxabutane-1,4-diyl, 2-chloro-2,3,3-trifluoro-1,4-dioxabutane-1,4-diyl, 2,2-difluoro-1,4-dioxabutane-1,4-diyl or 2,2-difluoro-1,3-dioxapropane-1,3-diyl, and, in the case where at least one of the radicals $R^3$, $R^5$ or $R^6$ represents trifluoromethyl, also hydrogen or chlorine, and, in the case where $R^1$ represents chlorine and at the same time $R^2$ represents fluorine, also chlorine, methoxy, ethoxy, propoxy or butoxy.

2. Process for the preparation of oxadiazine derivatives of the formula I

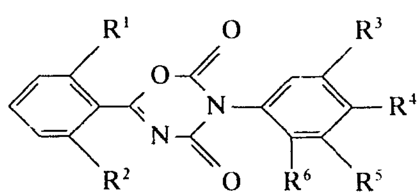

(I)

in which

$R^1$ represents fluorine, chlorine, bromine, iodine, methyl or ethyl,
$R^2$ represents hydrogen, fluorine, chlorine, bromine or iodine,
$R^3$, $R^5$ and $R^6$ are identical or different and represent hydrogen, chlorine, methyl or trifluormethyl and
$R^4$ represents tert.-butyl, tert.-pentyl, difluoromethyl, trifluoromethyl, chlorodifluoromethyl, difluoromethoxy, trifluoromethoxy, chlorodifluoromethoxy, 1,1,2,2-tetrafluoroethoxy, 2-chloro-1,1,2-trifluoroethoxy, trifluoromethylthio or chlorodifluoromethylthio, or, together with $R^3$, 2,2,4,4-tetrafluoro-1,3-dioxabutane-1,4-diyl, 2,2,3-trifluoro-1,4-dioxabutane-1,4-diyl, 2-chloro-2,3,3-trifluoro-1,4-dioxabutane-1,4-diyl, 2,2-difluoro-1,4-dioxabutane-1,4-diyl or 2,2-difluoro-1,3-dioxapropane-1,3-diyl, and, in the case where at least one of the radicals $R^3$, $R^5$ or $R^6$ represents trifluoromethyl,

also hydrogen or chlorine, and, in the case where $R^1$ represents chlorine and at the same time $R^2$ represents fluorine, also chlorine, methoxy, ethoxy, propoxy or butoxy,

characterised in that substituted benzoyl isocyanates of the formula (II)

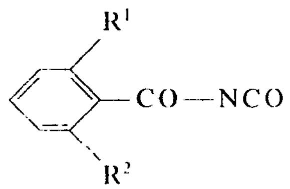

(II)

in which
$R^1$ and $R^2$ have the meaning indicated above,
are reacted with substituted phenyl isocyanates of the formula (III)

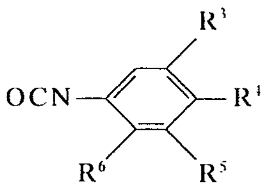

(III)

in which
$R^3$, $R^4$, $R^5$ and $R^6$ have the meaning indicated above,
if appropriate using inert diluents.

3. Agents for combating pests, characterised in that they contain compounds of the formula I.
4. Insecticides according to Claim 3, characterised in that they contain compounds of the formula I.
5. Use of compounds of the formula I for combating pests, in particular insects.
6. Process for the preparation of agents for combating pests, characterised in that compounds of formula I are mixed with extenders and/or surface-active agents.

**Revendications**

1. Dérivés oxadiaziniques de formule I:

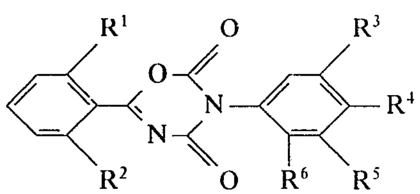

(I)

dans laquelle:

$R^1$ est le fluor, le chlore, le brome, l'iode, le groupe méthyle ou le groupe éthyle,
$R^2$ est l'hydrogène, le fluor, le chlore, le brome ou l'iode,
$R^3$ $R^5$ et $R^6$ sont égaux ou différents et représentent l'hydrogène, le chlore, le groupe méthyle ou le groupe trifluorométhyle,
$R^4$ représente le groupe tertio-butyle, tertio-pentyle, difluorométhyle, trifluorométhyle, chlorodifluorométhyle, difluorométhoxy, trifluorométhoxy, chlorodifluorméthoxy, 1,1,2,2-tétrafluoréthoxy, 2-chloro-1,1,2-trifluoréthoxy, trifluorométhylthio, chlorodifluorométhylthio ou forme avec $R^3$ le groupe 2,2,4,4-tétrafluoro-1,3-dioxabutane-1,4-diyle, 2,2,3-trifluoro-1,4-dioxabutane-1,4-diyle, 2-chloro-2,3,3-trifluoro-1,4-dioxabutane-1,4-diyle, 2,2-difluoro-1,4-dioxabutane-1,4-diyle ou 2,2-difluoro-1,3-dioxapropane-1,3-diyle et — au cas où au moins l'un des restes $R^3$, $R^5$ ou $R^6$ est le reste trifluorométhyle — également l'hydrogène ou le chlore, ainsi que — au cas où $R^1$ est le chlore et $R^2$ représente en même temps le fluor — également le chlore, le groupe méthoxy, le groupe éthoxy, le groupe propoxy ou le groupe butoxy.

2. Procédé de production de dérivés oxadiaziniques de formule I:

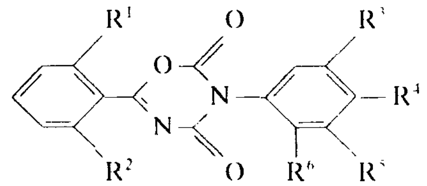

(I)

dans laquelle:

$R^1$ est le fluor, le chlore, le brome, l'iode, le groupe méthyle ou le groupe éthyle,
$R^2$ est l'hydrogène, le fluor, le chlore, le brome ou l'iode,
$R^3$, $R^5$ et $R^6$ sont égaux ou différents et représentent l'hydrogène, le chlore, le groupe méthyle ou le groupe trifluorométhyle,
$R^4$ représente le groupe tertio-butyle, tertio-pentyle, difluorométhyle, trifluorométhyle, chlorodifluorométhyle, difluorométhoxy, trifluorométhoxy, chlorodifluorométhoxy, 1,1,2,2-tétrafluoréthoxy, 2-chloro-1,1,2-trifluoréthoxy, trifluorométhylthio, chlorodifluorométhylthio ou forme avec $R^3$ le groupe 2,2,4,4-tétrafluoro-1,3-dioxabutane-1,4-diyle, 2,2,3-trifluoro-1,4-dioxabutane-1,4-diyle, 2-chloro-2,3,3-trifluoro-1,4-dioxabutane-1,4-diyle, 2,2-difluoro-1,4-dioxabutane-1,4-diyle ou 2,2-difluoro-1,3-dioxapropane-1,3-diyle et — au cas où au moins l'un des restes $R^3$, $R^5$ ou $R^6$ est le reste trifluorométhyle — également l'hydrogène ou le chlore, ainsi que — au cas où $R^1$ est le chlore et $R^2$ représente en même temps le fluor — également le chlore, le groupe méthoxy, le groupe éthoxy, le groupe propoxy ou le groupe butoxy,

caractérisé en ce qu'on fait réagir des benzoylisocyanates substitués de formule (II):

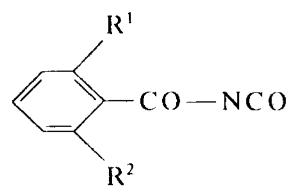

(II)

dans laquelle:
$R^1$ et $R^2$ ont la définition indiquée ci-dessus,
avec des phénylisocyanates substitués de formule (III):

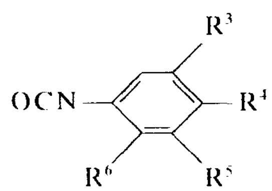

(III)

dans laquelle:
$R^3$, $R^4$, $R^5$ et $R^6$ ont la définition indiquée ci-dessus,
en utilisant éventuellement des diluants inertes.

3. Pesticides, caractérisés par une teneur en composés de formule I.

4. Insecticides suivant la revendication 3, caractérisés par une teneur en composés de formule I.

5. Utilisation de composés de formule I dans la lutte contre des parasites, notamment des insectes.

6. Procédé de préparation de pesticides, caractérisé en ce qu'on mélange des composés de formule I avec des diluants et/ou des agents tensio-actifs.